(19) Europäisches Patentamt
European Patent Office
Office européen des brevets

(11) Veröffentlichungsnummer: **0 035 734 B1**

(12) **EUROPÄISCHE PATENTSCHRIFT**

(45) Veröffentlichungstag der Patentschrift: **21.09.83**

(51) Int. Cl.³: **C 07 C 121/80,** C 07 C 120/00, A 61 K 31/275

(21) Anmeldenummer: **81101499.2**

(22) Anmeldetag: **03.03.81**

(54) **Neue 1-(Acylamino-aryloxy-)2-hydroxy-3-alkinyl-aminopropane und Verfahren zu ihrer Herstellung.**

(30) Priorität: **08.03.80 DE 3009036**

(43) Veröffentlichungstag der Anmeldung: ***16.09.81 Patentblatt 81/37***

(45) Bekanntmachung des Hinweises auf die Patenterteilung: **21.09.83 Patentblatt 83/38**

(84) Benannte Vertragsstaaten: **AT BE CH DE FR IT LI LU NL SE**

(56) Entgegenhaltungen:
CH-A-546 224
CH-A-546 227
DE-A-2 309 887
DE-A-2 403 809

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, D-6507 Ingelheim am Rhein (DE)**

(72) Erfinder: **Köppe, Herbert, Dr., Neuweg 72, D-6507 Ingelheim/Rhein (DE)**
Erfinder: **Kummer, Werner, Dr., *Georg-Scheuing-Strasse 15, D-6507 Ingelheim/Rhein (DE)***
Erfinder: **Stähle, Helmut, Dr., Rotweinstrasse 23, *D-6507 Ingelheim/Rhein (DE)***
Erfinder: **Muacevic, Gojko, Dr., In der Dörrwiese 13, D-6507 Ingelheim/Rhein (DE)**
Erfinder: **Traunecker, Werner, Dr., Birkenweg 1, D-6531 Münster-Sarmsheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue 1-(Acylaminoaryloxy)-2-hydroxy-3-alkinylaminopropane und Verfahren zu ihrer Herstellung

Die Erfindung betrifft neue Verbindungen der Formel I:

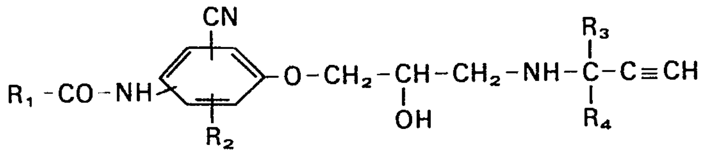
(I)

in der

$R_1$ einen Äthyl- oder Isopropylrest bedeutet,

$R_2$ ein Wasserstoff- oder Halogenatom, eine Alkyl- oder Alkoxygruppe mit 1 bis 4 C-Atomen oder die zweiwertigen Gruppen $-CH=CH-CH=CH-$ oder $CH_{2n}$ (n = ganze Zahl von 3 bis 5) mit Bindung der freien Valenzen in o-Stellung zueinander,

$R_3$ Wasserstoff oder einen Alkylrest mit 1 bis 3 C-Atomen,

$R_4$ einen Alkylrest mit 1 bis 3 C-Atomen oder zusammen mit $R_3$ die Gruppe $CH_{2p}$ (p = ganze Zahl von 4 bis 6),

ihre Säureadditionssalze, die Verwendung dieser Verbindungen in Arzneimitteln sowie ihre Herstellung.

Die neuen Verbindungen können auf folgende Weise hergestellt werden:

a) Umsetzung einer Verbindung der allgemeinen Formel II:

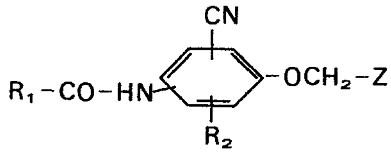
(II)

in der $R_1$ und $R_2$ wie in Formel I definiert sind und Z die Gruppe 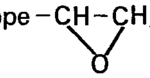 oder $-CHOH-CH_2-Hal$ (Hal = Halogen) bedeutet, mit einem Amin der allgemeinen Formel III:

$$NH_2-CR_2R_4-C\equiv CH \qquad (III)$$

in der $R_3$ und $R_4$ die in Formel I angegebenen Bedeutungen haben.

b) Hydrolyse einderivats der allgemeinen Formel IV:

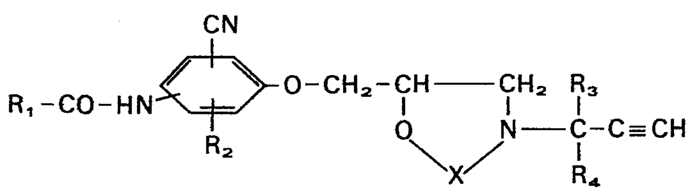
(IV)

in der $R_1$ bis $R_4$ wie in Formel I definiert sind und X eine $-CO-$, $-CH_2-$ oder $-CH-$Niederalkylgruppe bedeutet, beispielsweise mit Natron- oder kalilauge in Wasser oder in einer Alkohol/Wasser-Mischung.

c) Umsetzung einer Verbindung der allgemeinen Formel V:

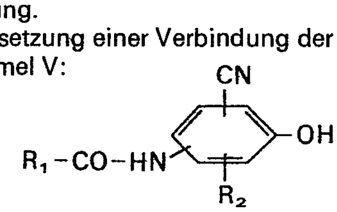
(V)

in welcher $R_1$ und $R_2$ die oben genannte Bedeutung haben, bzw. eines Salzes dieses Phenols, mit einem Azetidinolderivat der allgemeinen Formel VI:

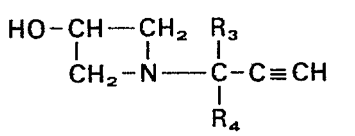
(VI)

in welcher $R_3$ und $R_4$ die oben genannte Bedeutung haben in wasserfreiem Medium.

Die Oxazolidinone der Formel IV (d.h. Verbindungen, bei denen X = CO darstellt) sind beispielsweise ausgehend von den Epoxiden der Formel II herstellbar, indem man letztere mit einem (aus Chlorameisensäureäthylester und einem Amin der Formel III darstellbaren) Urethan der Formel VII:

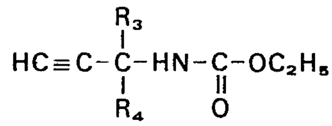
(VII)

in der $R_3$ und $R_4$ die obenbezeichneten Bedeutungen haben umsetzt.

Die Ausgangsphenole der allgemeinen Formel V und die Azetidinole der allgemeinen Formel VI können nach literaturbekannten Methoden [letztere s. beispielsweise „Chem. pharm. Bull." (Japan), vol. 22 (7), 1974, S. 1490] hergestellt werden.

Die erfindungsgemässen Verbindungen besitzen ein asymmetrisches C-Atom an der CHOH-Gruppe und kommen daher als Racemat wie auch in Form der optischen Antipoden vor. Letztere können ausser durch Racematentrennung mit üblichen Hilfssäuren wie Dibenzoyl- (bzw. Di-p-Tolyul) D-Weinsäure oder D-3-Bromcampher-8-sulfonsäure auch durch Einsetzen von optisch aktivem Ausgangsmaterial erhalten werden.

Die erfindungsgemässen 1-Phenoxy-2-hydroxy-3-alkinylaminopropane der allgemeinen Formel I können in üblicher Weise in ihre physiologisch verträglichen Säureadditionssalze überführt werden. Geeignete Säuren sind beispielsweise Salzsäure, Bromwasserstoffsäure, Schwefelsäure, Methansulfonsäure, Maleinsäure, Essigsäure, Oxalsäure, Milchsäure, Weinsäure oder 8-Chlortheophyllin.

Die Verbindungen der allgemeinen Formel I bzw. deren physiologisch verträgliche Säureadditionssalze haben im Tierversuch an Meerschweinchen wertvolle therapeutische, insbesondere β-adrenolytische Eigenschaften gezeigt und können daher beispielsweise zur Behandlung oder Prophylaxe von Erkrankungen der Herzkranzgefässe und zur Behandlung von Herzarrhythmien, insbesondere von Tachycardien, in der Humanmedizin eingesetzt werden. Auch die blutdrucksenkenden Eigenschaften der Verbindung sind therapeutisch interessant. Die Verbindungen haben gegenüber bekannten β-Rezeptorenblocker, z.B. dem strukturverwandten Handelsprodukt 1-(2-Acetyl-4-butyroylamino)phenoxy-2-hydroxy-3-isopropylaminopropan (Acebutolol) den Vorteil beträchtlich verminderter Toxizität, besserer aludrinantagonistischer Wirkung und hervorragender Organselektivität. Die Messung dieser Parameter erfolgte dabei nach den folgenden Modellvorschriften:

1. *Hemmung der Isoprenalintachycardie* (aludrinantagonistische Wirkung)

*Methode:* Hemmung der tachycarden Reaktion auf eine Standardosis Isoprenalin und Einfluss auf die basale Herzfrequenz durch steigende i.v. Dosen eines β-Adrenolytikums.

*Tiermaterial:* Meerschweinchen beiderlei Geschlechts mit Körpergewichten von 270-350 g, Gruppenhaltung, Standardkost und Wasser bis zum Versuchsbeginn *ad libitum*. 16 h vor dem Versuchsanfang Futterentzug.

*Narkose:* Äthyluräthan 1,75 g/kg als 20%ige Lösung intraperitoneal, gegebenenfalls wurde nachinjiziert.

*Präparation:* Kanülierung einer *Vena jugularis exterior* für intravenöse Injektionen: Einbinden einer Trachealkanüle und künstliche Beatmung subkutane Nadelelektroden zur Aufnahme des EKG, in der Regel Extremitätenableitung II, Registriergeschwindigkeit 25 mm/s; Rektalthermometer zur Kontrolle der Körpertemperatur, die mit einer Wärmelampe (Infrarotstrahler) auf 34-36°C mit Hilfe eine elektrischen Automatikeinrichtung konstant gehalten wird.

*Versuchsablauf:* Die Herzfrequenz wird durch Auszählen der R-Zacken im EKG bestimmt, jeweils aus einer 3-4 s langen Registrierzeit. Etwa 30 min nach der Präparation wird im Abstand von 2 min 5mal die normale Herzfrequenz gemessen und gemittelt. Anschliessend wird als adrenerges Stimulans 1 µg/kg Isoprenalin i.v. injiziert und danach 3 min lang alle 30 s die Herzfrequenz erneut registriert. Die Isoprenalininjektionen werden während des ganzen Versuches im Abstand von 30 min wiederholt. Bleibt die Spontanfrequenz etwa konstant und ist die tachycarde Reaktion auf die ersten 2-3 Isoprenalingaben gleichmässig, dann wird 15 min nach der letzten und 15 min vor der nächsten Isoprenalinreaktion die erste Dosis der Prüfsubstanz i.v. injiziert. Weitere in geometrischer Reihe ansteigende Dosen der Prüfsubstanz folgen in Abständen von 60 min bis eine markante hemmung der Isoprenalin-Tachycardie erreicht ist.

2. *Prüfung auf Cardioselektivität am wachen Meerschweinchen*

*Prinzip:* Nach der Methode von D. Dunlop und R.G. Shanks [brit. „J. Pharmacol." *32,* 201 (1968)] werden wache Meerschweinchen einer tötlichen Dosis eines Histaminaerosols ausgesetzt. Durch Vorbehandlung mit Isoprenalin werden die Tiere vor der letalen Wirkung des Histamins geschützt. Ein β-Adrenolytikum hebt die Isoprenalinwirkung auf, so dass der Schutz vor dem Histaminbronchospasmus verloren geht, falls es sich um eine nicht cardioselektive Substanz handelt. Zeigt eine am Herzen wirksame β-adrenolytische Substanz in diesem Test keinen Antagonismus gegen Isoprenalin, dann kann Cardioselektivität (für sog. $\beta_1$-Rezeptoren) angenommen werden.

*Tiermaterial:* Meerschweinchen beiderlei Geschlechts (6 Tiere pro Dosis), mit 350-400 g Körpergewicht, Gruppenhaltung, Standardfutter und Wasser bis zum Versuchsbeginn *ad libitum*. 16 h vor dem Versuchsanfang Futterentzug.

*Versuchsablauf:* Gruppen von je 6 Tieren (3 männliche + 3 weibliche) werden s.c. mit 5 oder mehr verschiedenen Dosen des β-Adrenolytikums behandelt. 15 min später erhalten sie 0,1 mg/kg Isoprenalin contralateral s.c. injiziert. Nach weiteren 15 min werden die Tiere in die zylindrische Kammer von 2 l Inhalt gesetzt, 45 s lang einem wässerigen Histaminaerosol (1,25 %ig) ausgesetzt und anschliessend wird die Mortalität ausgewertet.

*Auswertung:* Die Letalität wird gegen den Logarithmus der Dosis aufgetragen und die LD 50 nach J. Lithfield und F. Wilcoxon („J. Pharmacol. exp. Therap." *96,* 99-113, 1949) ermittelt. Mit der LD 50 aus diesem Versuch und der cardialen ED 50 aus dem Versuch der Hemmung der Isoprenalintachycardie (nark. Meerschweinchen) wird ein Selektivitätsquotient LD 50 / ED 50 gebildet. Eine Substanz wird als cardioselektiv angesehen, wenn der Quotient grösser als 1 ist.

Als wertvoll haben sich dabei insbesondere solche Verbindungen der allgemeinen Formel I herausgestellt, bei denen $R_3$ und $R_4$ jeweils eine Methylgruppe darstellen (substituierte p-Acylamino)-1-phenoxy-3-(2-methylbutinyl-3-amino-2)-2-propanole. Besonders wertvoll ist insbesondere das 1-(2-Cyano-4-isobutyroylamino)phenoxy-3-(2-methylbutinyl-3-amino-2)-2-propanol bzw. seine Salze. Diese Verbindung hat z.B. eine gegenüber dem Acebutolol etwa zwanzigfach stärkere aludrinantagonistische Wirkung.

Die Einzeldosis der erfindungsgemässen Substanzen liegt bei 1-300 mg, vorzugsweise 5-100 mg (oral) bzw. 1-20 mg (parenteral).

Die erfindungsgemässen Wirkstoffe können in die üblichen galenischen Anwendungsformen, wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffekts, wie Carboxypolymethylen, Carboxymethylcellulose, Celluloseacetatphthalat, oder Polyvinylacetat erhalten werden.

Die Tabletten können auch aus mehreren Schichten bestehen. Entsprechend können Dragées durch Überziehen von analog den Tabletten hergestellten Kernen mit üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffekts aus mehreren Schichten bestehen, wobei die oben bei den Tabletten erwähnten Hilfsstoffe verwendet werden können.

Säfte der erfindungsgemässen Wirkstoffe bzw. Wirkstoffkombinationen können zusätzlich noch ein Süssungsmittel, wie Saccharin, Cyclamat, Glycerin oder Zucker, sowie ein geschmackverbesserndes Mittel, z.B. Aromastoffe, wie Vanillin oder Orangenextrakt, enthalten. Sie können ausserdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete zäpfchen lassen sich beispielsweise durch Vermischen der dafür vorgesehenen Wirkstoffe bzw. Wirkstoffkombinationen mit üblichen Trägermitteln, wie Neutralfetten oder Polyäthylenglykol bzw. dessen Derivaten, herstellen.

Die erfindungsgemässen Verbindungen sind auch für die Kombination mit anderen pharmakodynamisch wirksamen Stoffen wie z.B. Coronardilatatoren, Sympathicomimetica, Herzglykosiden oder Tranquilizer geeignet.

Die folgenden Beispiele erläutern die Erfindung, ohne sie zu beschränken:

*A. Herstellungsbeispiele*

*Beispiel 1:*

*1(2-Cyano-4-n-propionylaminophenoxy)-3-(2-methylbutinyl-3-amino-2)-propanol*

7 g 1-(2-Cyano-4-n-propionylaminophenoxy)-2,3-epoxypropan werden in 100 ml Äthanol gelöst. Nach Zugabe von 7 g 2-Methylbutin-3-amin-2 wird 2 h unter Rückfluss zum Sieden erhitzt. Nach Abkühlen wird das Lösungsmittel abdestilliert, der Rückstand mit verdünnter HCl digeriert, zweimal mit je 150 ml Essigester ausgeschüttelt und die wässerige Phase abgetrennt. Sie wird mit Natronlauge alkalisch gestellt, die sich abscheidende Base in Essigester aufgenommen und die organische Phase mit $H_2O$ neutral gewaschen. Nach Trocknung über $Na_2SO_4$ wird sie eingeengt. Der verbleibende Rückstand wird aus Essigester unter Zugabe von Petroläther (Kp. 60°C) umkristallisiert.

Ausbeute: 3,4 g, Fp.: 138-139°C.

*Beispiel 2:*

*1-(2-Cyano-6-isobutyroylaminophenoxy)-3-(2-methylbutinyl-3-amino-2)propanoloxylat*

8,7 g (0,029 mol) 1-(2-Cyano-6-isobutyroylaminophenoxy)-2-hydroxy-3-chlorpropan werden in 80 ml Äther gelöst und 15,5 ml (0,147 mol) 2-Methylbutin-3-amin-2 zugegeben. Nach 1-stündigem Erhitzen unter Rückflusskühlung werden die flüchtigen Anteile im Vakuum abdestilliert. Der Rückstand wird in 65 ml verdünnter HCl aufgenommen, zweimal mit je 50 ml Essigester ausgeschüttelt und die wässerige Phase mit Natronlauge alkalisch gestellt. Die ausfallende Base wird dreimal mit je 75 ml Essigester extrahiert, die organische Phase über Natriumsulfat getrocknet, abgesaugt und im Vakuum eingeengt. Der Rückstand wird über eine Kieselgelsäule gereinigt.

Die die Reinsubstanz enthaltenden Fraktionen werden vereinigt und das Lösungsmittelgemisch im Vakuum abdestilliert. Der Rückstand wird in wenig Acetonitril gelöst und zu einer Lösung von 3,5 g Oxalsäure in 10 ml Acetonitril getropft. Nach Zugabe von etwas Äther kristallisiert das Oxalat farblos aus. Es wird aus Methanol unter Zugabe von Äther nochmals umkristallisiert.

Ausbeute: 2,4 g, Fp.: 195-196°C.

*Beispiel 3:*

*1-(2-Cyano-4-isobutyroylaminophenoxy)-3-(2-methylbutinyl-3-amino-2)-propanol*

3,1 g (0,015 mol) 2-Cyano-4-isobutyroylaminophenol werden mit 7,2 g (0,08 mol) Epichlorhydrin und 0,1 g Piperidin vermischt und 5 h bei 100°C gerührt. Nach Abdestillieren des überschüssigen Epichlorhydrins wird der Rückstand in 20 ml Äthanol gelöst, 4 ml 2-Methylbutin-3-amin-2 zugegeben und 3 h unter Rückfluss gekocht. Nach Abdestillieren des Lösungsmittels wird der verbleibende Rückstand mit verdünnter Salzsäure angesäuert und dreimal mit Äther extrahiert. Die wässerige Phase wird mit Natronlauge alkalisch gestellt und das sich abscheidende Amin mit Essigester ausgeschüttelt. Die organische Phase wird mit Wasser gewaschen, über $Na_2SO_4$ getrocknet und der Essigester abdestilliert. Der Rückstand wird in Äthanol gelöst, über Kohle abgesaugt und im Vakuum eingeengt. Der verbleibende Rückstand (3,3 g) wird in Äther digeriert, abgesaugt und mit Äther gewaschen. Die festen Anteile werden aus wenig Acetonitril (4 ml) umkristallisiert, mit Äther gewaschen und nochmals aus 4 ml Acetonitril umkristallisiert.
Ausbeute: 1,1 g, Fp.: 110-111°C.

Nach Einengen der vereinigten Mutterlaugen wird der Rückstand über eine Kieselgelsäule gereinigt. Durch Aufarbeiten der Fraktionen wird ein festes Kristallisat gewonnen, das aus Acetonitril umkristallisiert wird. Es werden weitere 1,3 g reine Substanz vom Fp.: 109,5-111°C erhalten.

*Beispiel 4:*

*1-(2-Cyano-4-isobutyroylaminophenoxy)-3-(1-äthinylcyclohexylamino)-2-propanol*

7,8 g (0,03 mol) 1-(2-Cyano-4-isobutyroylaminophenoxy)-2,3-epoxypropan werden in 80 ml äthanol gelöst und nach Zugabe von 7,5 g (0,06 mol) 1-Äthinylcyclohexylamin 1 h zum Sieden unter Rückfluss erhitzt. Das Lösungsmittel wird abdestilliert und der Rückstand mit verdünnter HCl angesäuert. Die wässerige Phase wird nach Ausschütteln mit Essigester durch Zugabe von NaOH alkalisch gestellt, wodurch basische Anteile ölig ausfallen. Sie werden in Essigester aufgenommen. Die organische Phase wird mit $H_2O$ gewaschen, über $MgSO_2$ getrocknet, eingeengt und der feste Rückstand zweimal aus Acetonitril umkristallisiert.
Ausbeute: 4,9 g, Fp.: 142-144°C.

*Beispiel 5:*

*1-(2-Cyano-4-isobutyroylaminophenoxy)-3-(3-äthylpentinyl-4-amino-3)-2-propanol*

7,8 g (0,03 mol) 1-(2-Cyano-4-isobutyroylaminophenoxy)-2,3-epoxypropan werden in 100 ml Äthanol mit 11,1 g (0,3 mol) 3-Äthylpentin-4-amin-3 eine Stunde am Rückfluss zum Sieden erhitzt. Nach Abdestillieren des Lösungsmittels wird der verbleibende Rückstand mit verdünnter HCl angesäuert, organische Neutralstoffe mit Äther extrahiert und die wässerige Phase mit NaOH alkalisch gestellt. Die ölig ausfallenden basischen Anteile werden in Essigester aufgenommen, die organische Phase wird mit Wasser gewaschen. Nach Trocknung über $Na_2SO_4$ wird der Essigester abdestilliert. Der verbleibende Rückstand wird zweimal aus Essigester unter Zugabe von Petroläther umkristallisiert.
Ausbeute: 5,8 g, Fp.: 98-99°C.

*Beispiel 6:*

*1-(2-Cyano-4-n-propionylaminophenoxy)-3-(2-methylbutinyl-3-amino-2)-2-propanol*

6,3 g (0,045 mol) 1-(2-Methylbutin-1-yl-3)-3-azetidinol werden in 30 ml Benzylakohol gelöst und unter stetigem Rühren 9,0 g (0,05 mol) 2-Cyano-4-n-propionylaminophenol und 100 mg KOH zugegeben. Im Stickstoffstrom wird das Gemisch 5 h auf 140°C erhitzt. Nach Abkühlung werden 70 ml Äther zugegeben. Durch mehrmaliges Ausschütteln mit verdünnter HCl werden die basischen Anteile extrahiert. Die wässerige Phase wird mit Äther gewaschen und mit NaOH alkalisch gestellt. Die ölig ausfallenden basischen Anteile werden in Essigester aufgenommen, mit $H_2O$ gewaschen und über $Na_2SO_4$ getrocknet. Nach Abdestillieren des Esters wird der Rückstand aus Essigester unter Zugabe von Petroläther umkristallisiert. Das Kristallisat wird noch zweimal auf gleiche Weise gereinigt.
Ausbeute: 4,1 g, Fp.: 137-139°C.

*Beispiel 7:*

*1-(2-Cyano-4-isobutyroylaminophenoxy)-3-(2-methylbutinyl-3-amino-2)-2-propanol*

4,06 g (0,01 mol) 3-(2-Methylbutin-3-amin-2)-5-(2-cyano-4-isobutyroylaminophenoxymethyl)oxazolidinon-2 werden in 25 ml Äthanol nach Zugabe von 2 g KOH in 5 ml $H_2O$ eine halbe Stunde unter Rückfluss zum Sieden erhitzt. Das Lösungsmittel wird sodann abdestilliert, der Rückstand mit $H_2O$ digeriert und mit Essigester ausgeschüttelt. Die organische Phase wird mit verdünnter HCl extrahiert, anschliessend die wässerige Phase mit Essigester gewaschen und mit NaOH alkalisch gestellt. Die ölig ausfallenden basischen Anteile werden in Essigester aufgenommen, mit Wasser gewaschen und über $Na_2SO_4$ getrocknet. Der wird abdestilliert. Der verbleibende Rückstand wird zweimal unter Zugabe von Petroläther umkristallisiert.
Ausbeute: 1,6 g, Fp.: 109-111°C.

*B. Formulierungsbeispiele*

1. *Tabletten*

| | |
|---|---|
| 1-(2-Cyano-4-isobutyroylaminophenoxy)-3-(2-methylbutinyl-3-amino-2)-2-propanol | 40,0 mg |
| Maisstärke | 164,0 mg |
| sek.-Calciumphosphat | 240,0 mg |
| Magnesiumstearat | 1,0 mg |
| | 445,0 mg |

Herstellung:
Die einzelnen Bestandteile werden intensiv miteinander vermischt und die Mischung in üblicher Weise granuliert. Das Granulat wird zu Tabletten von 445 mg Gewicht verpresst, von denen jede 40 mg Wirkstoff enthält.

Anstelle des in diesem Beispiel genannten Wirkstoffs können auch die Substanzen 1-(2-Cyano-4-isobutyroylaminophenoxy)-3-(1-äthinylcyclohexylamino)-2-propanol und 1-(2-Cyano-4-n-propionylaminophenoxy)-3-(2-methylbutinyl-3-amino-2-)-2-propanol in gleicher Menge verwendet werden.

2. *Gelatinekapseln*

Der Inhalt der Kapsel setzt sich wie folgt zusamment:

| | |
|---|---|
| 1-(2-Cyano-4-isobutyroylaminophenoxy)-3-(2-methylbutinyl-3-amino-2)-2-propanol | 25,0 mg |
| Maisstärke | 175,0 mg |
| | 200,0 mg |

Herstellung:

Die Bestandteile des Kapselinhalts werden intensiv vermischt und 200 mg-Portionen der Mischung werden in Gelatinekapseln geeigneter Grösse abgefüllt. Jede Kapsel enthält 25 mg des Wirkstoffs.

3. *Injektionslösung*

Die Lösung wird aus folgenden Bestandteilen hergestellt:

| | |
|---|---|
| 1-(2-Cyano-4-n-propionylaminophenoxy)-3-(2-methylbutinyl-3-amino-2)-2-propanol | 2,5 Teile |
| Natriumsalz der EDTA (Äthylendiamintetraessigsäure) | 0,2 Teile |
| dest. Wasser | ad 100,0 Teile |

Herstellung:

Der Wirkstoff und das EDTA-Salz werden in genügend Wasser gelöst und mit Wasser auf das gewünschte Volumen aufgefüllt. Die Lösung wird frei von suspendierten Partikeln filtriert und in 1 ccm-Ampullen unter aseptischen Bedingungen abgefüllt. Zuletzt werden die Ampullen sterilisiert und verschlossen. Jede Ampulle enthält 25 mg Wirkstoff.

4. *Depotdragées*

Kern

| | |
|---|---|
| (-)-1-(2-Cyano-4-isobutyroyl-phenoxy)-3-(2-methylbutinyl-3-amino-2)-2-propanol | 25,0 g |
| Carboxymethylcellulose (CMC) | 295,0 g |
| Stearinsäure | 20,0 g |
| Celluloseacetatphthalat (CAP) | 40,0 g |
| | 380,0 g |

Herstellung:

Der Wirkstoff, die CMC und die Stearinsäure werden intensiv gemischt und die Mischung in üblicher Weise granuliert, wobei man eine Lösung des CAP in 200 ml eines Gemisches aus Äthanol/Äthylacetat verwendet. Das Granulat wird dann zu 380 mg-Kernen verpresst, die in üblicher Weise mit einer zuckerhaltigen 5%igen Lösung von Polyvinylpyrrolidon in Wasser überzogen werden.

Jedes Dragée enthält 25 mg Wirkstoff.

5. *Tabletten*

| | |
|---|---|
| 1-(2-Cyano-4-isobutyroylaminophenoxy)-3-(3-äthylpentinyl-4-amino-3-)2-propanol | 35,0 g |
| 2,6-Bis-(diäthanolamino)-4,8-dipiperidinopyrimido-[5,4-d]-pyrimidin | 75,0 g |
| Milchzucker | 164,0 g |
| Maisstärke | 194,0 g |
| kolloidale Kieselsäure | 14,0 g |
| Polyvinylpyrrolidon | 6,0 g |
| Magnesiumstearat | 2,0 g |
| lösliche Stärke | 10,0 g |
| | 500,0 g |

Anstelle des in diesem Beispiel genannten β-adrenolytisch wirksamen Stoffs kann auch die Substanz 1-(2-Cyano-4-isobutyroylaminophenoxy)-3-(2-methylbutinyl-3-amino-)2-propanol in gleicher Menge verwendet werden.

Herstellung:

Der Wirkstoff wird zusammen mit dem Milchzucker, der Maisstärke, der kolloidalen Kieselsäure und dem Polyvinylpyrrolidon nach intensiver Durchmischung in üblicher Weise granuliert, wobei man eine wässerige Lösung der löslichen Stärke verwendet. Das Granulat wird mit dem Magnesiumstearat gemischt und zu 1000 Tabletten von je 500 mg Gewicht verpresst, die je 35 mg des ersten und 75 mg des zweiten Wirkstoffs enthalten.

**Patentansprüche**

für die Vertragsstaaten: BE, CH, DE, FR, IT, LI, LU, NL, SE

1. Neue Verbindungen der allgemeinen Formel (I):

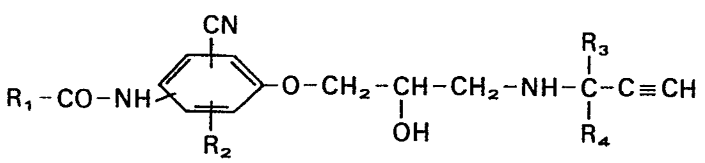

(I)

worin

$R_1$ einen Äthyl- oder Isopropylrest,

$R_2$ ein Wasserstoff- oder Halogenatom, eine Alkyl- oder Alkoxygruppe mit 1 bis 4 C-Atomen oder die zweiwertigen Gruppen

$$-CH=CH-CH=CH- \text{ oder } CH_{2n}$$

(n = ganze Zahl von 3 bis 5) mit Bindung der freien Valenzen in o-Stellung zueinander,

$R_3$ Wasserstoff oder einen Alkylrest mit 1 bis 3 C-Atomen und

$R_4$ einen Alkylrest mit 1 bis 3 C-Atomen oder zusammen mit $R_3$ die Gruppe $CH_{2p}$ (p = ganze Zahl von 4 bis 6)

bedeutet, und deren physiologisch verträgliche Säureadditionssalze.

2. Neue Verbindungen gemäss Anspruch 1 mit der allgemeinen Formel I, dadurch gekennzeichnet, dass $R_3$ und $R_4$ jeweils die Methylgruppe bedeuten, sowie deren physiologisch verträgliche Säureadditionssalze.

3. 1-(2-Cyano-4-isobutyroylamino)phenoxy-3-(2-methylbutinyl-3-amino-2)propanol bzw. seine Salze.

4. Pharmazeutische Zubereitungen, enthaltend Substanzen der allgemeinen Formel I bzw. ihre physiologisch verträglichen Säureadditionssalze in Kombination mit üblichen Hilfs- und/oder Trägerstoffen.

5. Pharmazeutische Präparate gemäss Anspruch 4, enthaltend Substanzen der allgemeinen Formel I bzw. ihre physiologisch verträglichen Säureadditionssalze in Kombination mit anderen pharmazeutischen Wirkstoffen sowie üblichen Hilfs- und/oder Trägerstoffen.

6. Verfahren zur Herstellung von Verbindungen gemäss Anspruch 1 mit der allgemeinen Formel (I):

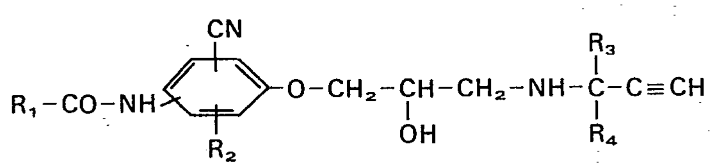
(I)

worin $R_1$ bis $R_4$ die oben genannte Bedeutung haben, dadurch gekennzeichnet, dass man

a) eine Verbindung der allgemeinen Formel (II):

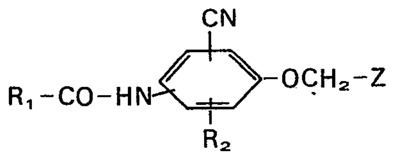
(II)

in der $R_1$ und $R_2$ wie in Formel I definiert sind und Z die Gruppe 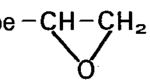 oder $-CHOH-CH_2-Hal$ (Hal = Halogen) bedeutet, mit einem Amin der allgemeinen Formel III:

$$NH_2-CR_2R_4-C\equiv CH \quad (III)$$

in der $R_3$ und $R_4$ wie in Formel I definiert sind, umsetzt, oder dass man

b) ein Oxazolidinderivat der allgemeinen Formel (IV):

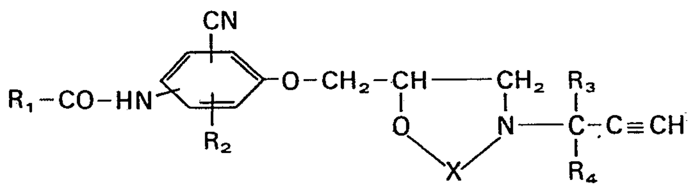
(IV)

in der $R_1$ bis $R_4$ wie in Formel I und X ein $-CO-$, $-CH_2-$ oder $-CHR$-Gruppe, worin R Niederalkyl ist, bedeutet, definiert sind, hydrolysiert, oder dass man

c) eine Verbindung der allgemeinen Formel (V):

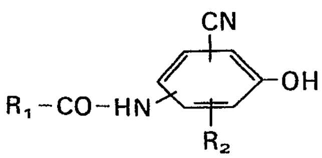
(V)

in welcher $R_1$ und $R_2$ wie in Formel I definiert sind, oder ein Salz dieser Verbindung, mit einem Azetidinolderivat der allgemeinen Formel (VI):

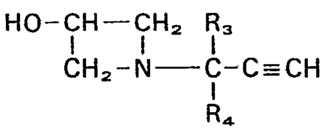
(VI)

in welcher $R_3$ und $R_4$ wie in Formel I definiert sind, umsetzt,

und die nach einem der Verfahren a bis c erhaltenen Verbindungen gewünschtenfalls in ihre Säureadditionssalze überführt.

7. Verfahren zur Herstellung von optisch aktiven Verbindungen der allgemeinen Formel I, dadurch gekennzeichnet, dass man

a) von optisch aktiven Ausgangsmaterialien ausgeht, oder dass man

b) die nach einem der Verfahren des Anspruchs 6 erhaltenen Verbindungen durch Umsetzung mit optisch aktiven Hilfssäuren in ihre diastereomeren Salze überführt und letztere durch fraktionierte Kristallisation auftrennt.

**Patentansprüche**
für den Vertragsstaat: AT

1. Verfahren zur Herstellung von Verbindungen mit der allgemeinen Formel (I):

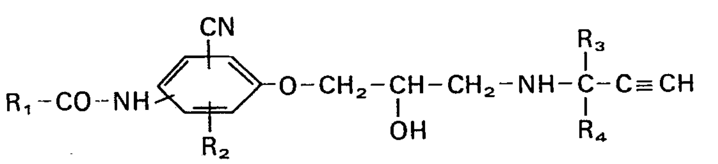
(I)

worin

$R_1$ einen Äthyl- oder Isopropylrest,

$R_2$ ein Wasserstoff- oder Halogenatom, eine Alkyl- oder Alkoxygruppe mit 1 bis 4 C-Atomen oder die zweiwertigen Gruppen

$$-CH=CH-CH=CH- \text{ oder } CH_{2n}$$

(n = ganze Zahl von 3 bis 5) mit Bindung der freien Valenzen in o-Stellung zueinander,

$R_3$ Wasserstoff oder einen Alkylrest mit 1 bis 3 C-Atomen und

$R_4$ einen Alkylrest mit 1 bis 3 C-Atomen oder zusammen mit $R_3$ die Gruppe $CH_{2p}$ (p = ganze Zahl von 4 bis 6)

bedeutet, und deren physiologisch verträgliche Säureadditionssalze, dadurch gekennzeichnet, dass man

a) eine Verbindung der allgemeinen Formel (II):

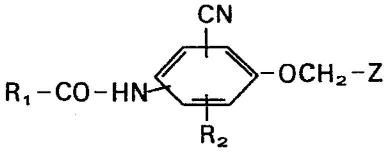 (II)

in der $R_1$ und $R_2$ wie in Formel I definiert sind und Z die Gruppe 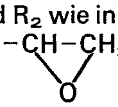 oder $-CHOH-CH_2-Hal$

(Hal = Halogen) bedeutet, mit einem Amin der allgemeinen Formel III:

$$NH_2-CR_3R_4-C\equiv CH \qquad (III)$$

in der $R_3$ und $R_4$ wie in Formel I definiert sind, umsetzt, oder dass man

b) ein Oxazolidinderivat der allgemeinen Formel (IV):

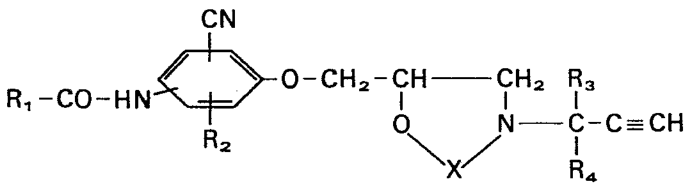 (IV)

in der $R_1$ bis $R_4$ wie in Formel I und X ein $-CO-$, $-CH_2-$ oder $-CHR$-Gruppe, worin R Niederalkyl ist, bedeutet, definiert sind, hydrolysiert, oder dass man

c) eine Verbindung der allgemeinen Formel (V):

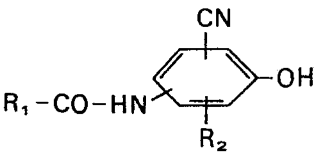 (V)

in welcher $R_1$ und $R_2$ wie in Formel I definiert sind, oder ein Salz dieser Verbindung, mit einem Azetidinolderivat der allgemeinen Formel (VI):

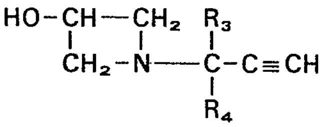 (VI)

in welcher $R_3$ und $R_4$ wie in Formel I definiert sind, umsetzt,

und die nach einem der Verfahren a bis c erhaltenen Verbindungen gewünschtenfalls in ihre Säureadditionssalze überführt.

2. Verfahren zur Herstellung von optisch aktiven Verbindungen der allgemeinen Formel I, dadurch gekennzeichnet, dass man

a) von optisch aktiven Ausgangsmaterialien ausgeht, oder dass man

b) die nach einem der Verfahren des Anspruchs 1 erhaltenen Verbindungen durch Umsetzung mit optisch aktiven Hilfssäuren in ihre diastereomeren Salze überführt und letztere durch fraktionierte Kristallisation auftrennt.

**Revendications**

pour les Etats contractants: BE, CH, DE, FR, IT, LI, LU, NL, SE

1. Nouveaux composés de formule générale:

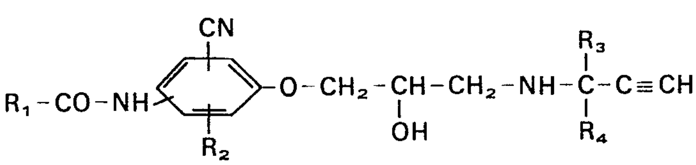 (I)

dans laquelle:

$R_1$ représente un radical éthyle ou isopropyle,

$R_2$ représente un atome d'hydrogène ou d'halogène, un groupe alcoyle ou alcoxy avec 1 à 4 atomes de C ou les groupes bivalents

$$-CH=CH-CH=CH- \text{ ou } CH_{2n}$$

(n = nombre entier de 3 à 5) avec liaison des valences libres en position o l'une par rapport à l'autre,

$R_3$ représente un atome d'hydrogène ou un radical alcoyle avec 1 à 3 atomes de C, et

$R_4$ représente un radical alcoyle avec 1 à 3 atomes de C ou ensemble avec $R_3$ le groupe $CH_{2p}$ (p = nombre entier de 4 à 6),

et leurs sels d'addition d'acides physiologiquement supportables.

2. Nouveaux composés selon la revendication 1 de formule générale (I), caractérisés en ce que $R_3$ et $R_4$ représentent chacun le groupe méthyle, ainsi que leurs sels d'addition d'acides physiologiquement supportables.

3. Le 1-(2-cyano-4-isobutyroylamino)phénoxy-3-(2-méthylbutynyl-3-amino-2)-propanol ou respectivement ses sels.

4. Compositions pharmaceutiques contenant des substances de formule générale (I) ou respectivement leurs sels d'addition d'acides physiologiquement supportables, en combinaison avec des adjuvants et/ou excipients habituels.

5. Préparations pharmaceutiques selon la revendication 4, contenant des substances de formule générale (I) ou respectivement leurs sels d'addition d'acides physiologiquement supportables en combinaison avec d'autres substances actives pharmaceutiques ainsi que des adjuvants et/ou excipients habituels.

6. Procédé de préparation de composés selon la revendication 1 ayant la formule générale:

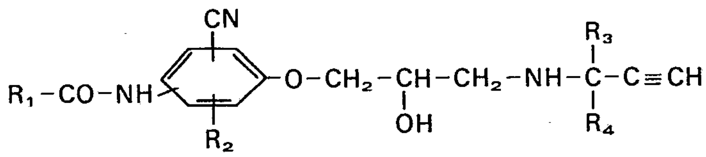 (I)

dans laquelle $R_1$ à $R_4$ ont la signification mentionnée plus haut, caractérisé en ce que

a) on fait réagir un composé de formule générale (II):

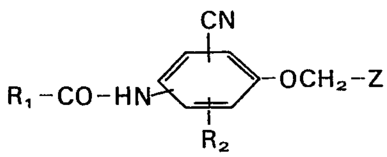 (II)

dans laquelle $R_1$ et $R_2$ sont définis comme dans la formule (I) et Z représente le groupe 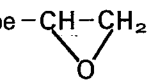 ou $-CHOH-CH_2-$hal (hal = halogène), avec une amine de formule générale:

$$NH_2-CR_3R_4-C\equiv CH \quad (III)$$

dans laquelle $R_3$ et $R_4$ sont définis comme dans la formule (I), ou en ce que

b) on hydrolyse un dérivé d'oxazolidine de formule générale (IV):

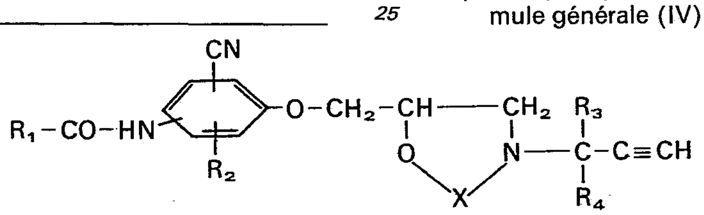 (IV)

dans laquelle $R_1$ à $R_4$ sont définis comme dans la formule (I) et X représente un groupe $-CO-$, $-CH_2-$ ou $-CHR$, où R est alcoyle inférieur, ou en ce que

c) on fait réagir un composé de formule générale (V):

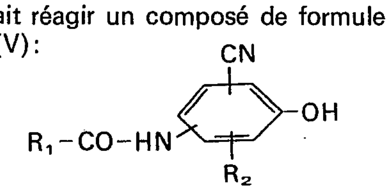 (V)

dans laquelle $R_1$ et $R_2$ sont définis comme dans la formule (I) (ou un sel de ce composé) avec un dérivé d'azétidinol de formule générale (VI):

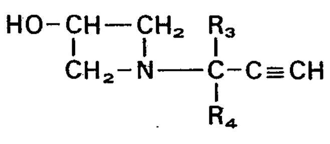 (VI)

dans laquelle $R_3$ et $R_4$ sont définis comme dans la formule (I),

et on transforme, si on le désire, les composés obtenus selon l'un des procédés a à c en leurs sels d'addition d'acides.

7. Procédé de préparation de composés optiquement actifs de formule générale (I), caractérisé en ce que

a) on part de matières de départ optiquement actives, ou en ce que

b) on transforme les composés obtenus selon l'un des procédés de la revendication 6 par réaction avec des acides adjuvants optiquement actifs, en leurs sels diastéréo-isomères et on sépare ces derniers par cristallisation fractionnée.

**Revendications**

pour l'Etat Contractant: AT

1. Procédé de préparation de composés ayant la formule générale:

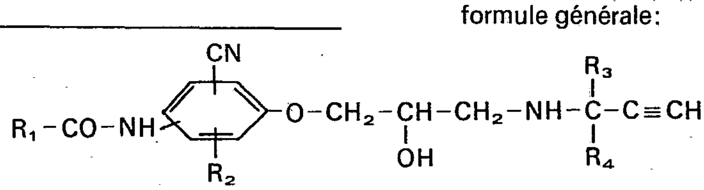 (I)

dans laquelle:

$R_1$ représente un radical éthyle ou isopropyle,

$R_2$ représente un atome d'hydrogène ou d'halogène, un groupe alcoyle ou alcoxy avec 1 à 4 atomes de C ou les groupes bivalents $-CH=CH-CH=CH-$ ou $(CH_2)_n$ (n = nombre entier de 3 à 5) avec liaison des valences libres en position o l'une par rapport à l'autre,

$R_3$ représente un atome d'hydrogène ou radical alcoyle avec 1 à 3 atomes de C, et

$R_4$ représente un radical alcoyle avec 1 à 3 atomes de C ou ensemble avec $R_3$ le groupe $CH_{2p}$ (p = nombre entier de 4 à 6),

et de leurs sels d'addition d'acides physiologiquement supportables, caractérisé en ce que

a) on fait réagir un composé de formule générale (II)

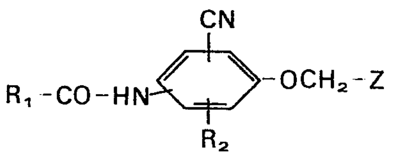 (II)

dans laquelle $R_1$ et $R_2$ sont définis comme dans la formule (I) et Z représente le groupe 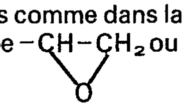 ou $-CHOH-CH_2-hal$ (hal = halogène), avec une amine de formule générale:

$$NH_2-CR_3R_4-CH{\equiv}CH \qquad (III)$$

dans laquelle $R_3$ et $R_4$ sont définis comme dans la formule (I), ou en ce que

b) on hydrolyse un dérivé d'oxazolidine de formule générale (IV):

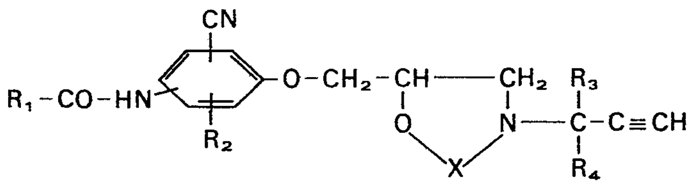 (IV)

dans laquelle $R_1$ à $R_4$ sont définis comme dans la formule (I) et X représente un groupe $-CO-$, $-CH_2-$ ou $-CHR$, où R est alcoyle inférieur, ou en ce que

c) on fait réagir un composé de formule générale (V):

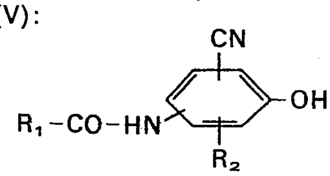 (V)

dans laquelle $R_1$ et $R_2$ sont définis comme dans la formule (I) (ou un sel de ce composé) avec un dérivé d'azétidinol de formule générale (VI):

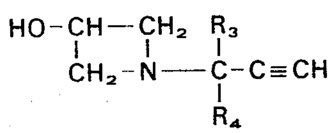 (VI)

dans laquelle $R_3$ et $R_4$ sont définis comme dans la formule (I), et en ce que, si on le désire, on transforme les composés obtenus selon l'un des procédés a à c en leurs sels d'addition d'acides.

2. Procédé de préparation de composés optiquement actifs de formule générale (I), caractérisé en ce que

a) on part de matières de départ optiquement actives, ou en ce que

b) on transforme les composés obtenus selon l'un des procédés de la revendication 1 par réaction avec des acides adjuvants optiquement actifs, en leurs sels diastéréo-isomères, et on sépare ces derniers par cristallisation fractionnée.

## Claims

for the Contracting States: BE, CH, DE, FR, IT, LI, LU, NL, SE

1. New compounds of general formula:

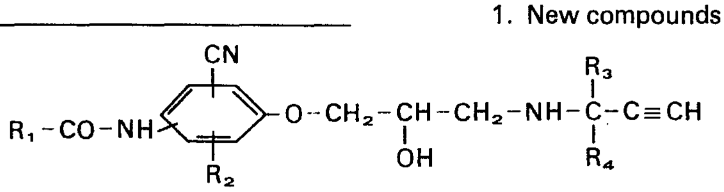 (I)

wherein:

$R_1$ represents an ethyl or isopropyl group;

$R_2$ represents a hydrogen or halogen atom, an alkyl or alkoxy group with 1 to 4 carbon atoms or the divalent groups

$$-CH{=}CH-CH{=}CH- \text{ or } CH_{2n}$$

(wherein n = an integer from 3 to 5) with the free valencies bonded in the o-position relative to one another,

$R_3$ represents hydrogen or an alkyl group with 1 to 3 carbon atoms, and

$R_4$ represents an alkyl group with 1 to 3 carbon atoms or, together with $R_3$, represents the group $(CH_2)_p$ (wherein p = an integer from 4 to 6),

and the physiologically acceptable acid addition salts thereof.

2. New compounds as claimed in claim 1 of general formula (I), characterised in that $R_3$ and $R_4$ each represent a methyl group, and the physiologically acceptable acid addition salts thereof.

3. 1-(2-Cyano-4-isobutyroylamino)phenoxy-3-(2-methylbutynyl-3-amino-2)-propanol and the salts thereof.

4. Pharmaceutical preparations, containing substances of general formula (I) or the physiologically acceptable acid addition salts thereof, in conjunction with conventional excipients and/or carriers.

5. Pharmaceutical preparations as claimed in claim 4, containing substances of general formula (I) or the physiologically acceptable acid addition salts thereof, in conjunction with other pharmaceutical active substances and conventional excipients and/or carriers.

6. Process for preparing compounds as claimed in claim 1 of general formula:

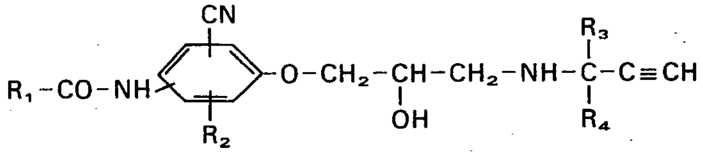 (I)

wherein:

$R_1$ to $R_4$ are as hereinbefore defined, characterised in that

a) a compound of general formula (II):

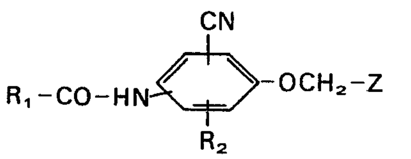 (II)

wherein $R_1$ and $R_2$ are defined as in formula (I), and Z represents the groupe 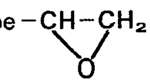 or $-CHOH-CH_2-Hal$ (wherein Hal = halogen), is reacted with an amine of general formula:

$$NH_2-CR_3R_4-C\equiv CH \qquad (III)$$

wherein $R_3$ and $R_4$ are defined as in formula (I), or

b) an oxazolidine derivative of general formula (IV):

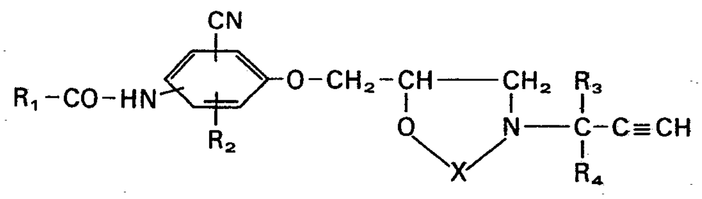 (IV)

wherein $R_1$ to $R_4$ are defined as in formula (I), and X represents a $-CO-$, $-CH_2-$ or $-CHR-$ group wherein R is lower alkyl, is hydrolysed, or

c) a compound of general formula (V)

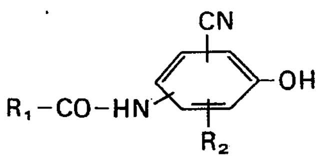 (V)

wherein $R_1$ and $R_2$ are defined as in formula (I) (or a salt of this compound) is reacted with an azetidinol derivative of general formula (VI):

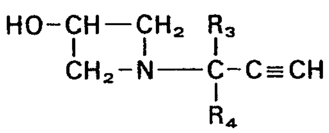 (VI)

wherein $R_3$ and $R_4$ are defined as in formula (I), and, if desired, the compounds obtained according to one of the processes a to c are converted into the acid addition salts thereof.

7. Process for preparing optically active compounds of general formula (I), characterised in that:

a) optically active starting materials are used, or
b) the compounds obtained according to one of the processes in claim 1 are converted into their diastereomeric salts by reacting them with optically active auxiliary acids and these salts are separated by fractional crystallisation.

**Claims**
for the Contracting State: AT

1. Process for preparing compounds of general formula:

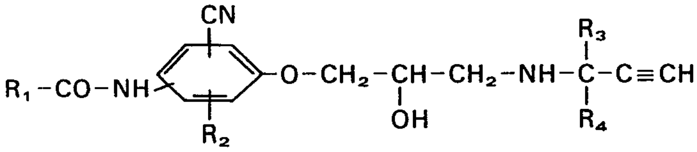 (I)

wherein:

$R_1$ represents an ethyl or isopropyl group,

$R_2$ represents a hydrogen or halogen atom, an alkyl or alkoxy group with 1 to 4 carbon atoms or the divalent groups

$$-CH=CH-CH=CH- \text{ or } CH_{2n}$$

(wherein n = an integer from 3 to 5) with the free valencies bonded in the o-position relative to one another,

$R_3$ represents hydrogen or an alkyl group with 1 to 3 carbon atoms, and

$R_4$ represents an alkyl group with 1 to 3 carbon atoms or, together with $R_3$, represents the group $CH_{2p}$ (wherein p = an integer from 4 to 6),

and the physiologically acceptable acid addition salts thereof, characterised in that:

a) a compound of general formula (II):

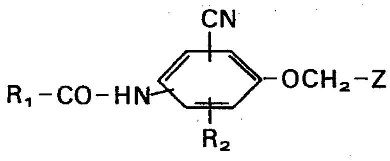 (II)

wherein $R_1$ and $R_2$ are defined as in formula (I) and Z represents the groupe 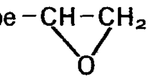 or $-CHOH-CH_2-Hal$ (wherein Hal = halogen), is reacted with an amine of general formula:

$$NH_2-CR_3R_4-C\equiv CH \quad (III)$$

wherein $R_3$ and $R_4$ are defined as in formula (I), or

b) an oxazolidine derivative of general formula (IV):

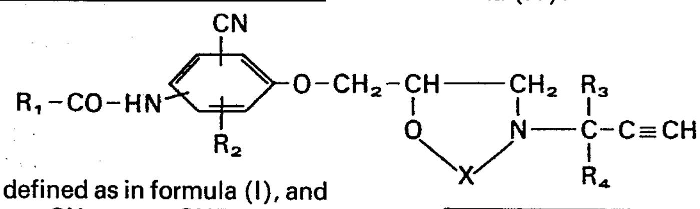 (IV)

wherein $R_1$ to $R_4$ are defined as in formula (I), and X represents a $-CO-$, $-CH_2-$ or $-CHR-$ group wherein R is lower alkyl, is hydrolysed, or

c) a compound of general formula (V):

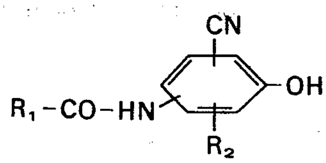 (V)

wherein $R_1$ and $R_2$ are defined as in formula (I) (or a salt of this compound) is reacted with an azetidinol derivative of general formula (VI):

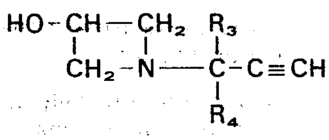 (VI)

wherein $R_3$ and $R_4$ are defined as in formula (I), and, if desired, the compounds obtained according to one of the processes a to c are converted into the acid addition salts thereof.

2. Process for preparing optically active compounds of general formula (I), characterised in that:

a) optically active starting materials are used, or
b) the compounds obtained according to one of the processes in claim 1 are converted into their diastereomeric salts by reacting them with optically active auxiliary acids and these salts are separated by fractional crystallisation.